# EUROPEAN PATENT APPLICATION

(11) **EP 2 455 877 A2**
(43) Date of publication of application: **23.05.2012**
(21) Application number: 12155076.8
(22) Date of filing: 29.06.2010
(51) Int. Cl.: G06F 19/00, A61B 5/145

(54) **Method for diabetes management**

(30) Priority: 30.06.2009 US 221840 P; 04.09.2009 US 239945 P
(62) Divisional of application: 10748159.0
(71) Applicant: Lifescan Scotland Limited, Inverness IV2 3ED (GB)
(72) Inventor: Jerdonek, Ronald, Alexandria VA 22314 (US); Longmuir, Alistair, Forres Morayshire IV36 1DL (GB); Raj, Siv, Beaconsfield HP9 1QW (GB); Macrae, Allan, Inverness IV2 3RG (GB); Weseley, Lisa, San Carlos CA 94070 (US); Stephens, Chris, Menlo Park CA 94025 (US)
(74) Representative: Brunner, John Michael Owen

(57) **Abstract**

Described herein are systems and methods to utilize factual information based on stored blood glucose data to allow greater insight into the management of diabetes of a user.

## Description

### BACKGROUND

This application claims the benefits of priority under 35 USC§ 119 and/or §120 from prior filed U.S. Provisional Application Serial Nos. 61/221,840 filed on June 30, 2009, and 61/239,945filed on September 4, 2009, which applications are incorporated by reference in their entirety into this application.

Glucose monitoring is a fact of everyday life for diabetic individuals. The accuracy of such monitoring can significantly affect the health and ultimately the quality of life of the person with diabetes. Generally, a diabetic patient measures blood glucose levels several times a day to monitor and control blood sugar levels. Failure to test blood glucose levels accurately and on a regular basis can result in serious diabetes-related complications, including cardiovascular disease, kidney disease, nerve damage and blindness. There are a number of electronic devices currently available which enable an individual to test the glucose level in a small sample of blood. One such glucose meter is the OneTouch® Profile™ glucose meter, a product which is manufactured by LifeScan.

In addition to glucose monitoring, diabetic individuals often have to maintain tight control over their lifestyle, so that they are not adversely affected by, for example, irregular food consumption or exercise. In addition, a physician dealing with a particular diabetic individual requires detailed information on the lifestyle of the individual to provide effective treatment or modification of treatment for controlling diabetes. Currently, one of the ways of monitoring the lifestyle of an individual with diabetes has been for the individual to keep a paper logbook of their lifestyle. Another way is for an individual to simply rely on remembering facts about their lifestyle and then relay these details to their physician on each visit.

The aforementioned methods of recording lifestyle information are inherently difficult, time consuming, and possibly inaccurate. Paper logbooks are not necessarily always carried by an individual and may not be accurately completed when required. Such paper logbooks are small and it is therefore difficult to enter detailed information requiring detailed descriptors of lifestyle events. Furthermore, an individual may often forget key facts about their lifestyle when questioned by a physician who has to manually review and interpret information from a hand-written notebook. There is no analysis provided by the paper logbook to distil or separate the component information. Also, there are no graphical reductions or summary of the information. Entry of data into a secondary data storage system, such as a database or other electronic system, requires a laborious transcription of information, including lifestyle data, into this secondary data storage. Difficulty of data recordation encourages retrospective entry of pertinent information that results in inaccurate and incomplete records.

Moreover, a diabetic individual often has to keep a plurality of devices on their person for diagnosis and treatment, for example both glucose level monitoring equipment and medication equipment. Hence, having to carry paper records of their lifestyle is an added unwanted burden and entry of data therein is very time consuming.

There currently exist a number of portable electronic devices that can measure glucose levels in an individual and store the levels for recalling or uploading to another computer for analysis. One such device is the Accu-Check™ Complete™ System from Roche Diagnostics, which provides limited functionality for storing lifestyle data. However, the Accu-Check™ Complete™ System only permits a limited selection of lifestyle variables to be stored in a meter. There is a no intelligent feedback from values previously entered into the meter and the user interface is unintuitive for an infrequent user of the meter.

### SUMMARY OF THE DISCLOSURE

In one embodiment, a method of providing analyte information of a user via an analyte meter is provided. The meter includes a microcontroller coupled to a memory, display, user interface buttons, and an analyte sensor. The method can be achieved by:
measuring with the analyte sensor for at least one analyte present in each of a plurality of physiological samples taken over a first predetermined time period; storing analyte values representative of the at least one analyte present in each of the plurality of physiological samples; determining whether a number of the stored analyte values over the first predetermined time period meets a minimum threshold; presenting, upon completion of a current analyte measurement and achievement of at least the minimum threshold of the number of stored analyte values, at least one of two selectable message headers comprising: (a) review of the stored analyte values over the predetermined time period; or (b) trend pattern of the stored analyte values; revealing, upon selection of the message header for review of stored analyte values, a message indicative of a distribution of stored analyte values over at least one of a predetermined range, a first threshold or a second threshold higher than the first threshold; and manifesting, upon selection of the message header for trend pattern, a message indicative of a trend of the stored values over a second predetermined time period.

In a further embodiment, a diabetes management system is provided that includes a blood glucose test strip and a blood glucose measurement device. The blood glucose test strip has an inlet port to receive a blood sample. The blood glucose measurement device includes a test strip port configured to receive the test strip and a microcontroller coupled to a memory, display screen, and user interface buttons so that upon completion of a current blood glucose measurement with the test strip, the display screen presents a value of the current blood glucose measurement and at least one of two selectable message headers on the display screen including a review of blood glucose values stored in the memory over a predetermined time period, or a trend pattern of the stored blood glucose values.

In yet another embodiment, an analyte test measurement device is provided that includes a housing, measurement sensor, microcontroller, memory, display screen and user interface buttons. The measurement sensor is disposed in the housing and configured to receive an analyte test strip. The microcontroller is coupled to the memory, display screen, user interface buttons and the measurement sensor which are mounted to the housing so that upon completion of a current analyte measurement of a physiological sample with an analyte test strip, the display screen presents the current analyte measurement and at least one of two selectable message headers screen including a review of analyte values stored in the memory over a predetermined time period or a trend pattern of the stored analyte values.

In yet a further embodiment, a method of providing analyte information of a user via an analyte meter is provided. The meter includes a microcontroller coupled to a memory, display, user interface buttons, and an analyte sensor. The method can be achieved by: measuring with the analyte sensor for at least one analyte present in each of a plurality of physiological samples taken over a first predetermined time period; storing analyte values representative of the at least one analyte present in each of the plurality of physiological samples; determining whether a number of the stored analyte values over the first predetermined time period meets a minimum threshold; querying the user, upon completion of a current analyte measurement and achievement of at least the minimum threshold of the number of stored analyte values, to select at least one of two selectable message headers comprising: (a) review of the stored analyte values over the predetermined time period; or (b) trend pattern of the stored analyte values; annunciating, upon selection of the message header for review of stored analyte values, a message indicative of a distribution of stored analyte values over at least one of a predetermined range, a first threshold or a second threshold higher than the first threshold; and annunciating, upon selection of the message header for trend pattern, a message indicative of a trend of the stored values over a second predetermined time period.

In yet a further embodiment, a method of providing analyte information of a user via an analyte meter is provided. The meter has a microcontroller coupled to a memory, display, user interface buttons, and an analyte sensor. The method can be achieved by:
measuring with the analyte sensor for at least one analyte present in each of a plurality of physiological samples taken over a first predetermined time period; reporting to the user at least one result from the measuring; storing analyte values representative of the at least one analyte present in each of the plurality of physiological samples; determining whether a number of the stored analyte values over the first predetermined time period meets a minimum threshold; upon determining that the number meets the minimum threshold,
evaluating the stored analyte values against one or more rules prestored in the meter to define a message having some of the stored data as part of the message to the user; and
manifesting the message to the user. The message can be the first message delivered to the user before any other messages.

In yet another embodiment, a diabetes management system is provided that includes a blood glucose test strip and a blood glucose measurement device. The blood glucose test strip has an inlet port to receive a blood sample. The blood glucose measurement device has a test strip port configured to receive the test strip and a microcontroller coupled to a memory, display screen, and user interface buttons so that upon completion of a current blood glucose measurement with the test strip, the display screen presents a value of the current blood glucose measurement and at least one of two selectable message headers on the display screen. The message headers include a review of blood glucose values stored in the memory over a predetermined time period, or a prompt to tag the current blood glucose measurement.

In a further embodiment, a method of providing analyte information of a user via a glucose meter is provided. The meter has a microcontroller coupled to a memory, display, user interface buttons, and a glucose sensor. The method can be achieved by: measuring a glucose concentration with the glucose sensor in each of a plurality of physiological samples taken over a first predetermined time period; automatically identifying, for a particular day, a low trend in glucose values that is detected on a particular day where at least one glucose concentration is below a second predetermined threshold in each of the last three days;automatically identifying, for a particular day, a high trend in glucose values that is detected on a particular day where at least one glucose concentration is above a first predetermined threshold in each of the last three days at a same time interval; and reporting to the user a plurality of dates where each date has only one corresponding message, the corresponding messages comprise either the high trend or the low trend, and if on a particular date both the high trend and the low trend are triggered, then only the low trend is reported to the user.

These and other embodiments, features and advantages will become apparent to those skilled in the art when taken with reference to the following more detailed description of the embodiments of the invention in conjunction with the accompanying drawings that are first briefly described here below.

### BRIEF DESCRIPTION OF THE DRAWINGS

The accompanying drawings, which are incorporated herein and constitute part of this specification, illustrate presently preferred embodiments of the invention, and, together with the general description given above and the detailed description given below, serve to explain features of the invention.

Figure 1 illustrates a diabetes management system that includes an analyte measurement and management device.

Figure 2 illustrates a top portion of an exemplary circuit board of the analyte measurement and management device for the device of Figure 1.

Figure 3 illustrates a bottom portion of the circuit board of the analyte measurement and management device.

Figures 4A and 4B illustrate user interface 400 that allows for communication of factual information of the user's blood glucose data.

Figure 4C illustrates another embodiment of a user interface 400C that allows for communication of factual information of the user's blood glucose data.

Figures 5A, 5B, and 5C illustrate additional menu selections for the diabetes patient.

Figure 6 illustrates an alternate user interface for messages relating to a seven-day review of blood glucose data, snapshot of the seven-day review and additional details of the review.

Figure 7 illustrates another alternate user interface for messages relating to blood glucose trends.

Figure 8 illustrates a system that allows for replication or embodiment of user interfaces described and illustrated herein for mobile communication devices.

### DETAILED DESCRIPTION OF ILLUSTRATIVE EMBODIMENTS

The following detailed description should be read with reference to the drawings, in which like elements in different drawings are identically numbered. The drawings, which are not necessarily to scale, depict selected exemplary embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. In addition, as used herein, the terms "patient," "host," "user," and "subject" refer to any human or animal subject and are not intended to limit the systems or methods to human use, although use of the subject invention in a human patient represents a preferred embodiment.

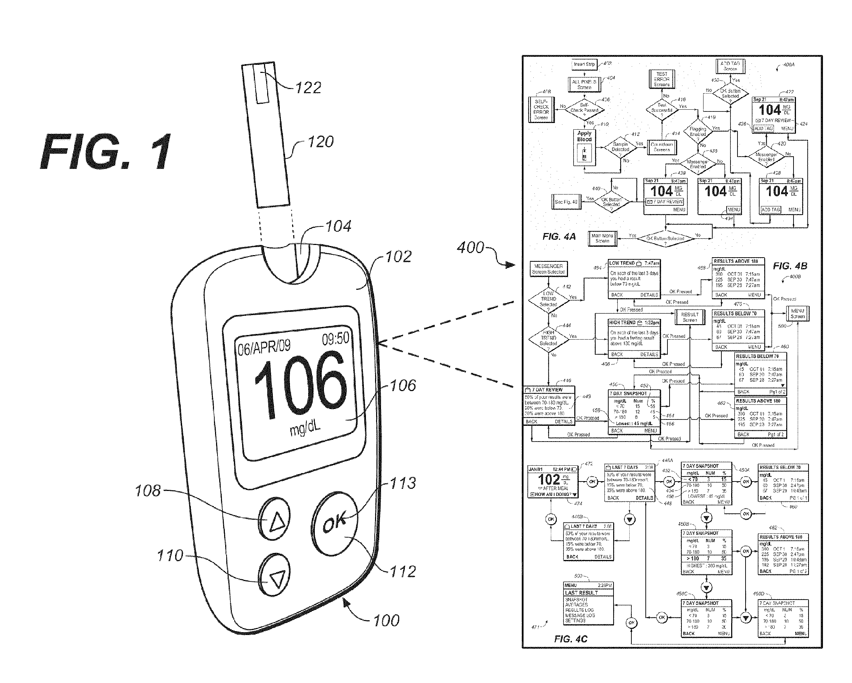

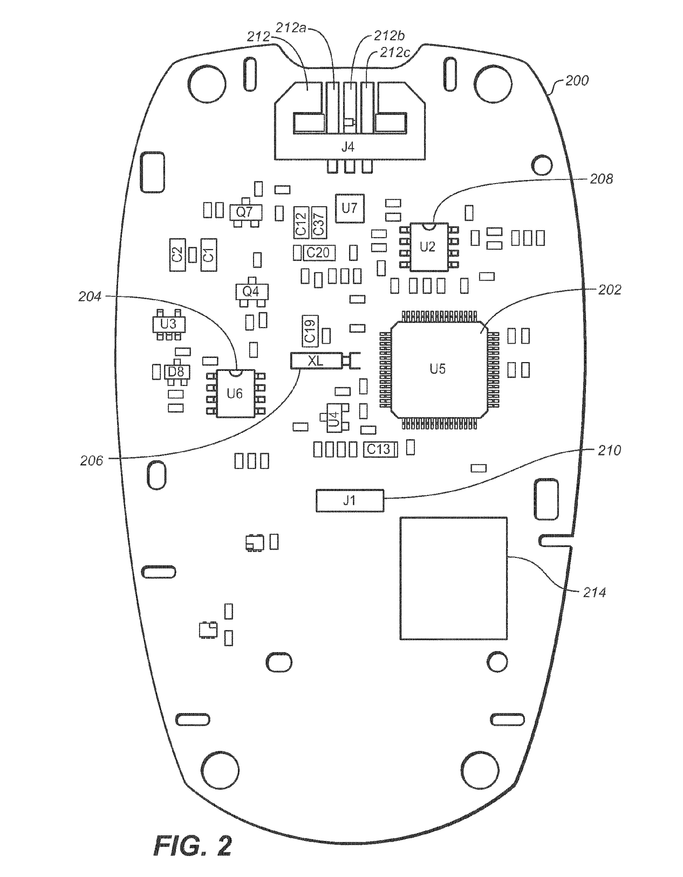

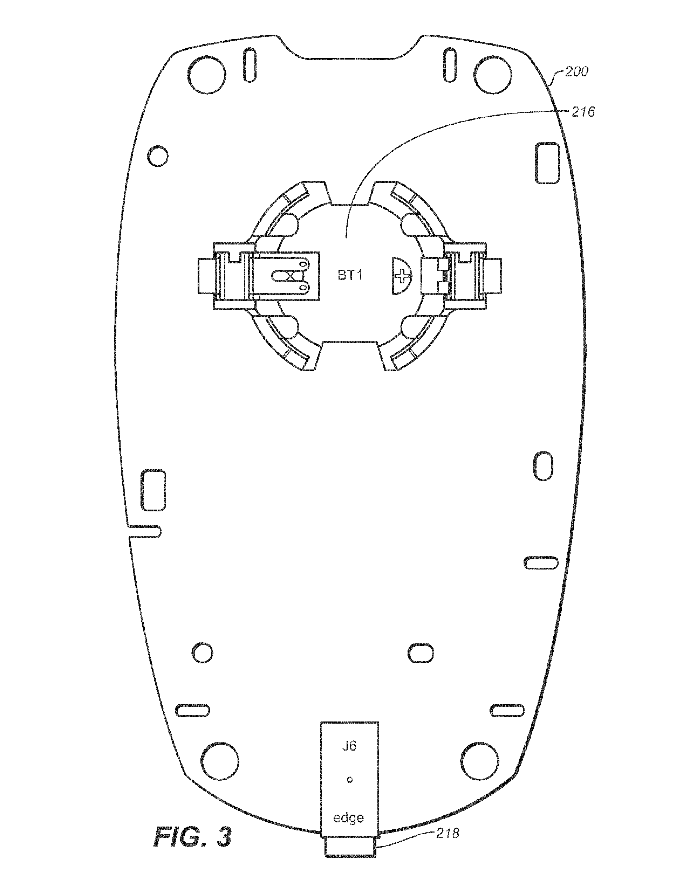

Figure 1 illustrates a diabetes management system that includes a blood glucose measurement unit 100 with a blood glucose test strip 120 having an inlet port 122 to receive a blood sample with an user interface 400 designed to assist the user in the management of diabetes. The blood glucose measurement device 100 includes a housing 102 with a test strip port 104 configured to receive the test strip and a display 106 along with user interface buttons 108, 110, and 112. Disposed inside housing 102 includes, as shown in Fig. 2, a circuit board 200 with a microcontroller 202 coupled to a memory 204, clock 206, operational amplifier 208, and display connector 210. The op-amp 208 and microcontroller 202 are operatively connected to a strip port connector 212 with contacts 212a, 212b, and 212b for mechanical contact with corresponding conductive tracks on the test strip 120. To facilitate communication with other data management devices, a wireless transceiver module 214 is provided to allow for bi-directional communication of data stored in the memory 204 of the unit 100. On the other side of circuit board 200, shown here in Fig. 3, a power source in the form of a battery 212 is provided. A data port 216 may also be provided. It should be noted that the unit 100 is preferably sized and configured to be handheld and the transceiver 214 can be for use with either or both of a short-range wireless network (e.g., BlueTooth or Wi-Fi and the like) or a longer range wireless network (e.g., GSM, CDMA, 3G and the like).

Microcontroller 202 can be electrically connected to strip port 212, operational amplifier circuit 208, first wireless module 214, display 106, non-volatile memory 204, clock 206, battery connector 216, data port 218, and user interface buttons (108, 110, and 112). Specifically, user interface buttons (108, 110, and 112) include a first user interface button 108, a second user interface button 110, and a third user interface button 112. User interface buttons (108, 110, and 112) include a first marking 109, a second marking 111, and a third marking 113, respectively, which allow a user to navigate through the user interface. Data entered can include values representative of analyte concentration, or in the context of the analyte concentration values coupled with information, which are related to the everyday lifestyle of an individual. Information, which is related to the everyday lifestyle, can include food intake, medication use, occurrence of health check-ups, and general health condition and exercise levels of an individual coupled to or "tagged" with the analyte concentration value of the user at specific time of the day or week.

Operational amplifier circuit 208 can be two or more operational amplifiers configured to provide a portion of the potentiostat function and the current measurement function. The potentiostat function can refer to the application of a test voltage between at least two electrodes of a test strip. The current function can refer to the measurement of a test current resulting from the applied test voltage to the test strip 120. The current measurement may be performed with a current-to-voltage converter. Microcontroller 202 can be in the form of a mixed signal microprocessor (MSP) such as, for example, the Texas Instrument MSP430F2419. The TI-MSP430F2419 can be configured to also perform a portion of the potentiostat function and the current measurement function. In addition, the MSP430F2419 can also include volatile and non-volatile memory. In another embodiment, many of the electronic components can be integrated with the microcontroller in the form of an application specific integrated circuit (ASIC).

Strip port 212 can be configured to form an electrical connection to the test strip. Display connector 210 can be configured to attach to display 106. Display 106 can be in the form of a liquid crystal display for reporting measured glucose levels, and for facilitating entry of lifestyle related information and for manipulation of graphical data, pictorial results and motion video. Display 106 may also include a backlight. Data port 218 can accept a suitable connector attached to a connecting lead, thereby allowing meter unit 100 to be linked to an external device such as a personal computer. Data port 218 can be any port that allows for transmission of data such as, for example, a serial, USB, or a parallel port. Clock 206 can be configured for measuring time and be in the form of an oscillating crystal. Battery connector 216 can be configured to be electrically connected to a power supply.

Referring to Fig. 1, test strip 120 can be in the form of an electrochemical glucose test strip. Test strip 120 can include one or more working electrodes and a counter electrode. Test strip 120 can also include a plurality of electrical contact pads, where each electrode is in electrical communication with at least one electrical contact pad. Strip port 212 can be configured to electrically interface to the electrical contact pads and form electrical communication with the electrodes of test strip 120. Test strip 120 can include a reagent layer that is disposed over at least one electrode. The reagent layer can include an enzyme and a mediator. Exemplary enzymes suitable for use in the reagent layer include glucose oxidase, glucose dehydrogenase (with pyrroloquinoline quinone co-factor, "PQQ"), and glucose dehydrogenase (with flavin adenine dinucleotide co-factor, "FAD"). An exemplary mediator suitable for use in the reagent layer includes ferricyanide, which in this case is in the oxidized form. The reagent layer can be configured to physically transform glucose into an enzymatic by-product and in the process generate an amount of reduced mediator (e.g., ferrocyanide) that is proportional to the glucose concentration value. The working electrode can then measure a concentration of the reduced mediator in the form of a current. In turn, meter unit 100 can convert the current magnitude into a glucose concentration value.

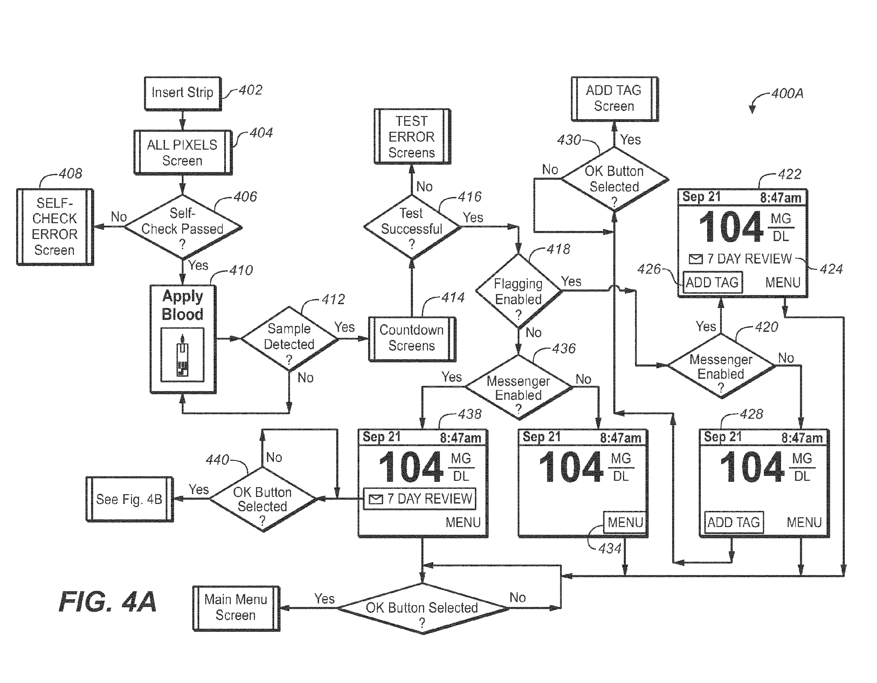

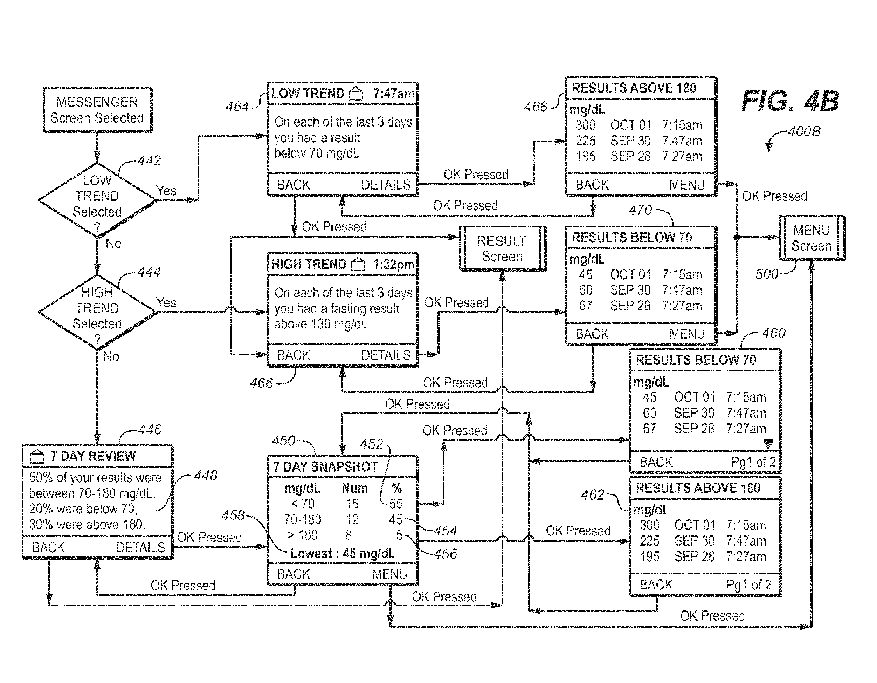

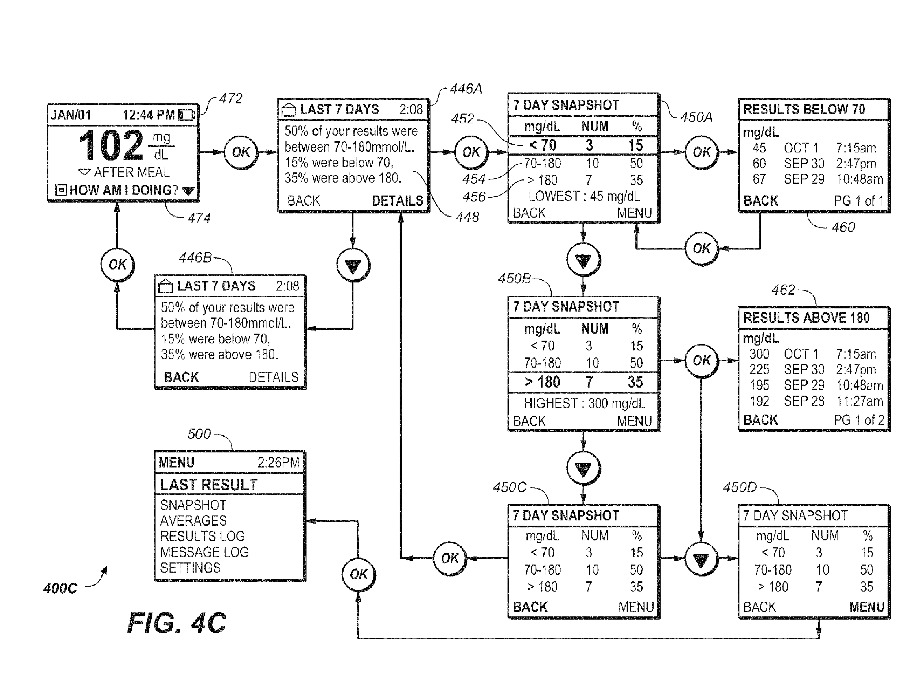

Figures 4A, 4B, and 4C illustrate a user interface 400A, 400B, 400C (collectively 400) that can be implemented with the aforementioned methods to provide insight for the diabetes user via the use of the meter unit 100. The insights or information provided to the user may be in the form of factual reporting and designed to be intuitive while sufficiently terse in fitting to the display 106. In one embodiment, programs and methods for conducting user interface 400 can be stored on non-volatile memory 204 of meter unit 100. Steps and instructions of user interface 400 can be displayed on display 106 of meter unit 100.

The user interface 400A can be activated upon the user carrying out a blood glucose measurement. Specifically, the meter 100 is turned on whenever the test strip 120 is inserted in step 402 with the conductive tracks into the test strip connector port 104. At step 404, the display 106 of the meter performs an all pixels screen and thereafter conducting a self-check test of all systems in step 406, which may result in an error screen 408 being displayed. In the event that there is no error in the system, the system is now ready to perform a blood glucose measurement by informing the user via the display with a representative pictorial representation of the strip and blood in screen 410. As the user deposit a blood sample on the sampling port 122 of the test strip 120 (Fig. 1), the op-amp provides an output voltage to the conductive tracks of the test strip that to detect whether a sample has been detected and whether a sufficient volume has been provided in step 412. Upon passing the sample detection test 412, the meter 100 counts down (and outputting the same via display 106 of a countdown timer) in step 414 the duration of the chemical reaction prior to the test meter 100 (via the microcontroller and op-amp) detecting a current output from the test strip. Assuming that there is no error detected in step 416, the detected current is thereafter converted into a blood glucose concentration displayed to the user, as shown in screen 404, as well as the date and time of the blood glucose measurement.

In step 418, it is determined whether the user has the ability to flag the just-completed blood glucose measurement. If step 418 returns a yes, the meter determines whether messages to the users have been enabled in step 420. Where the message function has been enabled in 420, the meter causes the display 106 to display in screen 422 the blood glucose measured along with the date, time along the top of display 106 and the availability of a message in the form of a seven day review 424 and a prompt 426 in the form of a dialog rectangle (which may be flashing to further emphasize the prompt) for the user to add contextual information to the blood glucose in the form of a tag to the displayed blood glucose measurement.

Where the messenger function has not been enabled in decision diamond 420, the display 106 shows screen 428 in which only the flag prompt is provided. In either of screen 422 or screen 428, the meter detects whether the OK button 112 is depressed by the user in step 430. Where the user has elected not to flag the result, the user may utilize the up arrow button 108 to move the prompt to highlight the message dialog 432 or the menu selection 434.

At decision diamond 418, if flagging has not been enabled but messenger has been enabled as determined at diamond 436 then the display 106 is configured to provide screen 438 allow the user to immediately select the "7 DAY REVIEVV" prompt 432 with one push of the OK button as determined at diamond 440.

Referring to Fig. 4B for continuation of user flow 400A, designated here as 400B, decision diamond 442 determines whether messages for low blood glucose trend have been selected and if not then decision diamond 444 decides whether messages for high blood glucose trend have been selected and if not the display 106 presents screen 446 with a seven day summary message 448 indicative of a distribution of stored analyte values, which may be provided in text, audio, visual or combination of audiovisual to identify a percentage of stored analyte values within a first distribution range, a percentage of stored analyte values, if any, lower than a minimum in the first distribution range and a percentage, if any, higher than a maximum in the first distribution range. Preferably, at least one message includes the following textual information: "_% of your result were between 70-180 mg/dL"; "_% were below 70"; or "_% were above 180." In the event that the user or caretaker(s) is interested in more details from the summary message, the user interface 400B is configured to allow selection of the details of the summary with only one actuation of the OK button. As used herein, "textual" refers to alphanumeric text that describes in a short factual description of the analyzed data rather than simply a presentation of all data, which in most cases would confuse the user.

Upon pressing of the OK button, meter 100 can provide for a snapshot of the last seven days via visual or audio messages. Preferably, the message includes a factual display of data which has been sorted to indicate a distribution of stored analyte values that includes a first distribution range 454, a second distribution range 452 with a value smaller than a minimum in the first distribution range, and a third distribution range 456 with a value greater than a maximum in the first distribution range, actual number of measurements having values within respective first, second and third distribution ranges, and a percentage of the measurements within respective first, second and third distribution ranges. The message of screen 450 may also include data regarding the lowest blood glucose measured during this 7 day review period.

To provide further insight into the data collected by the meter, the user interface 400B can be configured to allow the user or caretaker to highlight each of the distribution ranges 452 and 456 to allow for detailed review. In particular, when distribution range 452 is highlighted by actuation of one of the button 108 or 110 and actuation of the OK button, screen 460 can be provided as a message in text form or in audio visual form (not shown) to the user or caretaker. Screen 460 is indicative of a distribution of stored analyte values below a second predetermined threshold that includes measured values and the dates and times corresponding to the respective measured values. Alternatively, the user can select the distribution range 456 in screen 450 to allow for screen 462. Screen 462 is indicative of a distribution of stored analyte values above a first predetermined threshold that includes measured values and the dates and times corresponding to the respective measured values. It should be clear that any of the screens described herein this application can be provided alternatively in text, audio or a combination of audio and visual information.

Returning back to decision diamond 442 of Fig. 4B, in the event that messages for low blood glucose trend have been selected previously by the user or via customized set up, screen 464 is provided if there are sufficient data to indicate a low trend. Alternatively, in the event that the high blood glucose trend has been selected, screen 466 is provided if there are sufficient data to support a report of such trend. That is, depending on the actual data collected in meter 100, a message can be provided which is indicative of a trend of the stored values over a second predetermined time period that includes at least message for one of an increasing trend or decreasing trend in the measured values of the analyte.

For even more details, the user can press the OK button at screen 464 which will allow screen 468 to be provided. Alternatively, the user can press the OK button at screen 466 which will allow screen 470 to be accessed. That is, respective detailed messages are provided which are indicative of a trend of the stored values and may include a table having the date, time, and measured analyte value at such date and time in respective screens 468 and 470. Thereafter, the user interface 400 (including parts A and B) is configured to return the user back to the menu screen 500 upon depression of the OK button.

Figure 4C illustrates another embodiment of a user interface 400C, where the messenger has already been enabled, that allows for communication of factual information of the user's blood glucose data. Display 106 is configured to provide screen 472 that allows the user to immediately select a message header "HOW AM I DOING?" query 474 with one push of the OK button.

Referring to Figure 4C, display 106 presents screen 446A with a seven day summary message 448 indicative of a distribution of stored analyte values, which may be provided in text, audio, visual or combination of audiovisual to identify a percentage of stored analyte values within a first distribution range, a percentage of stored analyte values, if any, lower than a minimum in the first distribution range and a percentage, if any, higher than a maximum in the first distribution range. Preferably, at least one message includes the following textual information: "_% of your result were between 70-180 mg/dL"; "_% were below 70"; or "_% were above 180." In the event that the user or caretaker(s) is interested in more details from the summary message, the user interface 400C is configured to allow selection of the details of the summary with only one actuation of the OK button. As used herein, "textual" refers to alphanumeric text that describes in a short factual description of the analyzed data rather than simply a presentation of all data, which in most cases would confuse the user.

Note that screen 446A has an option labeled as "DETAILS" highlighted and that screen 446B has an option labeled as "BACK" highlighted. Screen 446A can move to screen 446B by pressing user interface button 110. Also, screen 446B can move to screen 472 by pressing the OK button.

Upon pressing of the OK button at screen 446A, meter 100 can provide for a snapshot 450A of the last seven days via visual or audio messages, as illustrated in Figure 4C. Preferably, the message includes a factual display of data which has been sorted to indicate a distribution of stored analyte values that includes a first distribution range 454, a second distribution range 452 with a value smaller than a minimum in the first distribution range, and a third distribution range 456 with a value greater than a maximum in the first distribution range, actual number of measurements having values within respective first, second and third distribution ranges, and a percentage of the measurements within respective first, second and third distribution ranges. The message of screens 450A or 450B may also include data regarding the lowest blood glucose measured during this 7 day review period.

Referring back to Figure 4C, screens 450A, 450B, 450C, and 450D correspond to a screen having different portions highlighted, which are a second distribution range 452, a third distribution range 456, the label "BACK," and the label "MENU," respectively. A user can navigate through screens 450A, 450B, 450C, and 450D by using user interface buttons 108 and 110. Screen 450C can move back to screen 446A by pressing the OK button. Screen 450D can move back to menu screen 500 by pressing the OK button.

To provide further insight into the data collected by the meter, the user interface 400C can be configured to allow the user or caretaker to highlight each of the distribution ranges 452 and 456 to allow for detailed review, as illustrated in snapshots 450A and 450B, respectively. In particular, when distribution range 452 is highlighted by actuation of one of the button 108 or 110 and actuation of the OK button, screen 460 can be provided as a message in text form or in audio visual form to the user or caretaker. Screen 460 is indicative of a distribution of stored analyte values below a second predetermined threshold that includes measured values and the dates and times corresponding to the respective measured values. The second predetermined threshold may correspond to a minimum in the first distribution range. Alternatively, the user can select the distribution range 456 in screen 450B to allow for screen 462. Screen 462 is indicative of a distribution of stored analyte values above a first predetermined threshold that includes measured values and the dates and times corresponding to the respective measured values. The first predetermined threshold may correspond to a maximum in the first distribution range.

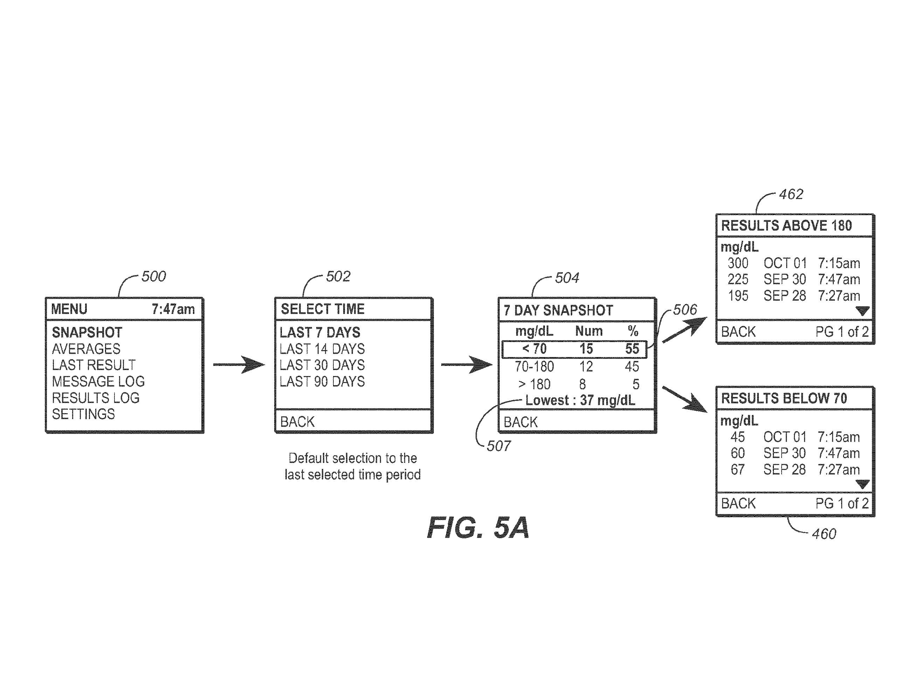

Referring to Figure 5A, menu screen 500 provides a menu of choices in reviewing of blood glucose data. The choices in screen 500 may include, for example, a snapshot of results over a variety of time periods (e.g., 3, 5, 7, 14, 21, 30, 60, 90 days, or any suitable time period); averages over the variety of time periods; the last result; a log of messages provided to the user or caretaker(s) and setting for the meter device. Of particular interest is the ability for the user to select the choices by graphically emphasizing the selected choice in screen 502 or a highlight box 506 in screen 504. In either case, the selection can be changed by pressing the up or down button. Selection can be by pressing the OK button such as in screen 504 resulting in screen 462 or screen 460. Dynamic message 507 of screen 504 can also be presented that are relevant to the stored analyte data and the highlight box 506 such as, for example, insulin doses, exercise, food, medication and the like. That is, dynamic message 507 is presented as a message that may change even though screen 504 remains the same and which message is dependent on the range of glucose selected in highlighted box 506 or criteria designated as more relevant to the user. In this example, the dynamic message 507 is provided as data regarding the lowest blood glucose measured during this 7 day review period.

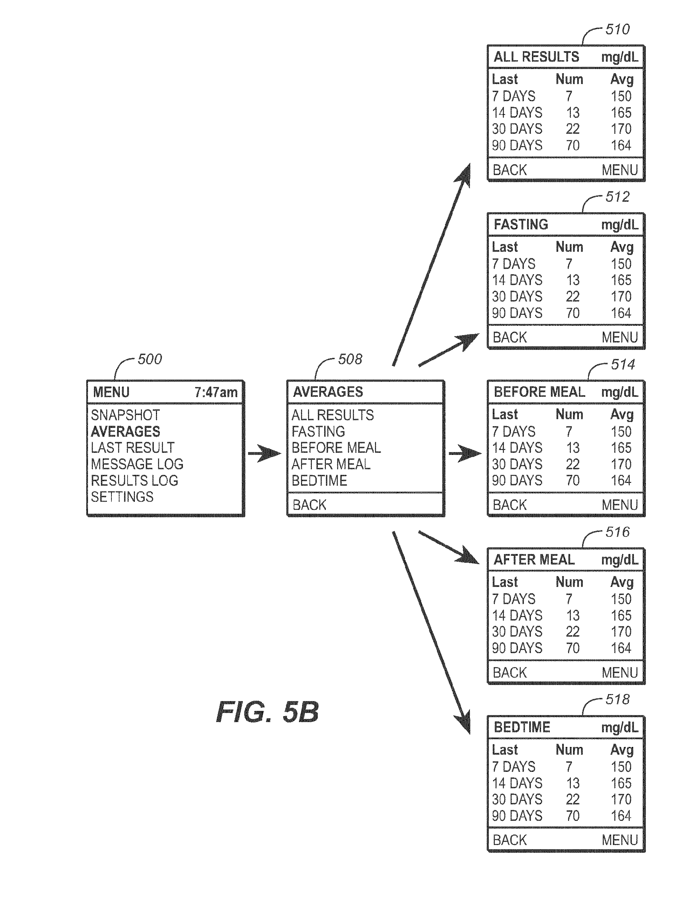

Referring to Figure 5B, at menu screen, the user or caretaker may decide to review the averages across a whole spectrum of defined thresholds such as, for example in screen 508, averages over defined time periods (e.g., 7, 14, 30, or 90 days) for all results in screen 510; averages over defined time periods (e.g., 7, 14, 30, or 90 days) in screen 512 for only for results flagged as "fasting" results; averages over defined time periods (e.g., 7, 14, 30, or 90 days) in screen 514 only for results flagged as "before meal"; averages over defined time periods (e.g., 7, 14, 30, or 90 days) in screen 516 only for result flagged as "after meal"; or averages over defined time periods (e.g., 7, 14, 30, or 90 days) in screen 518 only for results flagged as measured at "bedtime".

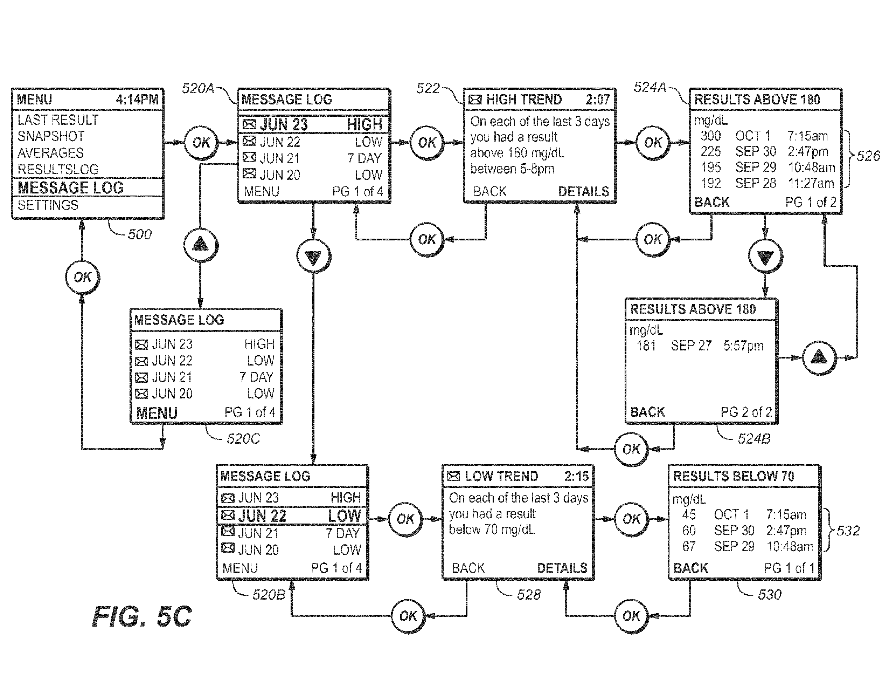

Referring to Figure 5C, at menu screen 500, the user or caretaker may decide to review the message log that includes an associated message for a particular day, where each day may include one or more glucose measurements. Upon selecting "MESSAGE LOG" on menu screen 500, display 106 can present screen 520A that includes a plurality of message log dates and associated messages. In an embodiment, there can be three types of associated messages such as, for example, "HIGH," "LOW," and "7 DAY," as illustrated in screen 520A. In an embodiment, there can be only one associated message with a particular message log date for simplicity. If more than one associated message can be associated with a particular message log date, then only the associated message with the highest priority will be shown. The associated messages can be displayed with the following priority where "LOW" has the highest priority, "HIGH" has the next highest priority, and "7 DAY" has the lowest priority. The following will describe in more detail the associated messages "LOW," "HIGH," and "7 DAY."

The associated message "LOW" represents a low trend in glucose values that was detected on a particular day. The meter automatically identifies a low trend when there was a glucose concentration below a second predetermined threshold (e.g., 70 mg/dL) in each of the last three days. Note the low trend has the highest priority because hypoglycemic glucose concentrations can cause imminent and serious physical harm to the user if undetected.

The associated message "HIGH" represents a high trend in glucose values that was detected on a particular day. The meter automatically identifies a high trend when there was a glucose concentration above a first predetermined threshold (e.g., 180 mg/dL) in each of the last three days at the same time interval. In an embodiment, a time interval may be a three hour interval where a day is divided into eight discrete intervals. Note that the high trend has the next highest priority because hyperglycemic glucose concentrations can also cause serious physical harm, but is generally not as imminent a danger as hypoglycemia. Hyperglycemia tends to cause long-term complications, whereas, hypoglycemia can potentially cause immediate death.

The associated message "7 DAY" indicates that the user had selected the message header "HOW AM I DOING?" query 474 (see Figure 4C) for a particular day. The "7 DAY" associated message is shown if user had selected query 474 and the meter did not recognize either a high trend or low trend for the particular day. Note that the message header query 474 may also be referred to simply as a prompt. And as used herein, the two terms "query" and "prompt" are synonymous with one another.

Referring back to Figure 5C, a user can highlight a particular day such as a first day (e.g., JUN 23, as illustrated in 520A), a second day (e.g., JUN 22, as illustrated in 520B), or highlight a "MENU" selection (see 520C) using user interface buttons 108 and 110.

Referring back screen 520A of Figure 5C, pressing the OK button would select the message log date of JUN 23, which would cause screen 522 to display a high trend message. The high trend message would indicate that there was at least one glucose concentration above a first predetermined threshold (e.g., 180 mg/dL) in each of the last three days at the same time interval (e.g., between 5 to 8 pm). The user can further find additional details by pressing the OK button which would take the user to screen 524A which provides for a breakdown 526 (e.g., measured value, date, and time) of the occurrence of the data above the high blood glucose threshold. Additional breakdown information can be found on subsequent screen 524B using user interface buttons 108 and 110.

In an embodiment, the high trend message in screen 522 can also include flagging information where the user has entered such information. For instance, an appropriate type of flag (e.g., fasting, before meal, after meal, or bedtime) can also be displayed with the high trend message where in each of the last three days at the same time interval (e.g., between 5 to 8 pm) there are at least three glucose concentrations that all have the same type of flag and were all above a first predetermined threshold (e.g., 180 mg/dL). In another embodiment, an appropriate type of flag (e.g., fasting, before meal, after meal, or bedtime) can be displayed with the high trend message where there is at least one glucose concentration in each of the last three days, at any time interval, that all have the same type of flag and were all above a first predetermined threshold (e.g., 180 mg/dL).

Referring back screen 520B of Figure 5C, pressing the OK button would select the message log date of JLTN 22, which would cause screen 528 to display a low trend message. The low trend message would indicate that there was a glucose concentration below a second predetermined threshold (e.g., 70 mg/dL) in each of the last three days. The user can further find additional details by pressing the OK button which would take the user to screen 530 which provides for a breakdown 532 (e.g., measured value, date, and time) of the occurrence of the data below the low blood glucose threshold.

In an embodiment, the low trend message in screen 528 can also include flagging information where the user has entered such information. For instance, an appropriate type of flag (e.g., fasting, before meal, after meal, or bedtime) can also be displayed with the low trend message where in each of the last three days there is at least one glucose concentrations that all have the same type of flag and were all below a second predetermined threshold (e.g., 70 mg/dL).

In yet another embodiment, the low trend message in screen 528 can also specify whether there is at least one glucose concentration in each of the last three days that is below a second predetermined threshold (e.g., 70 mg/dL), that occurs at the same time interval (e.g., between 5 to 8 pm). In yet another embodiment, the low trend message in screen 528 can also specify whether there is at least one glucose concentration in each of the last three days that is below a second predetermined threshold (e.g., 70 mg/dL), that occurs at the same time interval (e.g., between 5 to 8 pm), and that has the same type of flag (e.g., fasting, before meal, after meal, or bedtime).

It is noted that the flagging process can be implemented manually by the user or automatically by the meter 100. In one embodiment, a fasting flag can be recommended based on the time, the day, and/or past user testing patterns. For example, if a user had selected the "fasting" flag at 7 am multiple times, then the meter will suggest that the same "fasting" flag for the next reading performed at around 7 am. In one embodiment, the predictive process may require that at least "n" glucose readings be performed during the same time period with a fasting flag. The minimum number of glucose readings having a matching flag during a particular time interval can be adjusted by the user or health care provider. For example, the sub-routine can require that three of the last five glucose readings for a particular time period have the fasting flag. A time period can be defined as a two hour period, but optionally can be adjusted by the user or health care provider. Once a user is presented with a recommended fasting flag, the user has the option to override the suggestion or accept it.

In another embodiment, a fasting flag can be recommended if the test is the first test of the day. A microprocessor and clock of the meter unit 100 can determine the first time in which it is turned on. In another embodiment, a fasting flag can be recommended based on a user inputted meal time schedule. Before performing a glucose test, the user may use the user interface of meter unit 100 to input the meal time schedule. Alternatively, a default meal time schedule can be saved to a memory portion of the meter at the factory. If the glucose test is performed before a first meal of the day, then the fasting flag can be recommended. In another embodiment, a fasting flag can be recommended if the glucose test is the first test of the day and is in the morning such as, for example, between 6 am to 10 am. A morning time interval can be defined by the user or be a default setting set when the meter is manufactured.

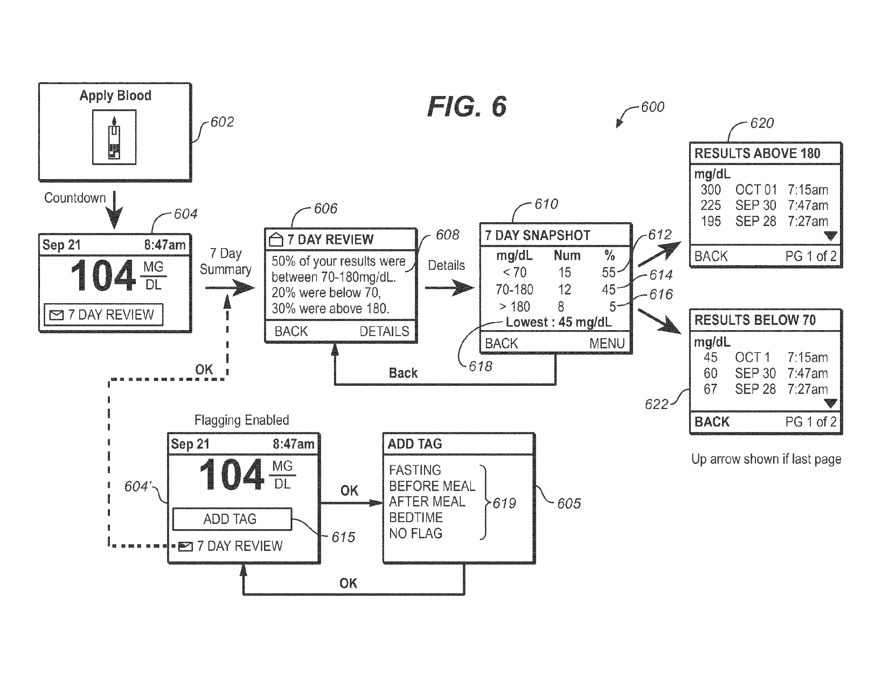

Referring to Figure 6, yet another user interface 600 can be implemented whenever there is a sufficient number of blood glucose measurements conducted over a predetermined period of time. Assuming that such threshold requirement is met upon the user conducting a blood glucose measurement at screen 602, the meter provides a display of the result of the measurement at screen 604 while highlighting that a seven (7) day review is available. Upon the user pressing the OK button with the message "7 DAY REVIEW" highlighted, the meter 100 presents screen 606 with a seven day summary message 608 indicative of a distribution of stored analyte values, which may be provided in text, audio, visual or combination of audiovisual to identify a percentage of stored analyte values within a first distribution range, a percentage of stored analyte values, if any, lower than a minimum in the first distribution range and a percentage, if any, higher than a maximum in the first distribution range. Preferably, at least one message 608 includes the following text: "_% of your result were between 70-180 mg/dL"; "_% were below 70."; or "_% were above 180." In the event that the user or caretaker(s) is interested in more details from the summary message, the user interface 600 is configured to allow selection of the details of the summary with only a single actuation of the OK button.

Upon pressing of the OK button, meter 100 can provide for a snapshot of the last seven days via visual or audio messages. Preferably, the snapshot message 610 includes a factual display of data which has been sorted to indicate a distribution of stored analyte values that includes a first distribution range 612, a second distribution range 614 with a value smaller than a minimum in the first distribution range, and a third distribution range 616 with a value greater than a maximum in the first distribution range, actual number of measurements having values within respective first, second and third distribution ranges, and a percentage of the measurements within respective first, second and third distribution ranges. Dynamic message 618 of screen 610 can also be presented that are relevant to the stored analyte data such as, for example, insulin doses, exercise, food, medication and the like. The dynamic message 618 is presented dynamically depending on the range of glucose selected or the range of glucose designated as more relevant to the user. In this example, the dynamic message 618 is provided as data regarding the lowest blood glucose measured during this 7 day review period.

To provide further insight into the data collected by the meter, the user interface 600 can be configured to allow the user or caretaker to highlight each of the distribution ranges 612 and 616 to allow for detailed review. In particular, when distribution range 616 is highlighted by actuation of one of the button 108 or 110 and actuation of the OK button, screen 620 can be provided as a message in text form or in audio visual form (not shown) to the user or caretaker. Screen 620 is indicative of a distribution of stored analyte values above a first predetermined threshold that includes measured values and the dates and times corresponding to the respective measured values. Alternatively, the user can select the distribution range 612 in screen 610 to output screen 622. Screen 622 is indicative of a distribution of stored analyte values below a second predetermined threshold that includes measured values and the dates and times corresponding to the respective measured values. Where flagging by the user has been enabled in the set up of the user interface, the user interface 600 is configured to prompt the user at screen 604' to enter a tag or flag by highlighting the query 615 in screen 604' which would take the user to screen 605 to allow the user to select and add the appropriate flag (e.g., fasting, before meal, after meal, bedtime, or no flag) 619. After the appropriate flag has been added, the screen returns to screen 604'. Thereafter, the user interface continues from screen 604' to screen 606 and screen 610. It should be clear that any of the screens described herein this application can be provided alternatively in text, audio or a combination of audio and visual information.

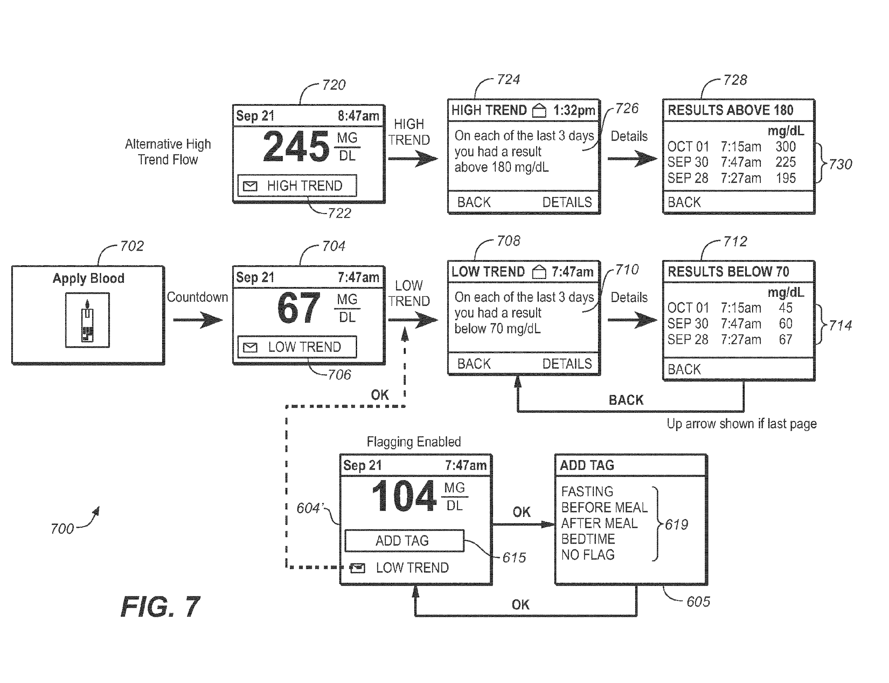

Referring to Figure 7, another user interface 700 can be implemented whenever there is a sufficient number of blood glucose measurements conducted over a predetermined period of time. Assuming that such threshold requirement is met upon the user conducting a blood glucose measurement at screen 702 then the factual messaging of user interface 700 can be utilized. Depending on whether the stored data is indicative of a low blood glucose trend or a high blood glucose trend, the user interface 700 can provide certain messages whenever the stored data meet certain rules. For example, a message is provided to the user whenever a blood glucose reading at any time in the preceding 3 days is below a second predetermined threshold, such as, for example 70 mg/dL or at any value from 50 to 90 mg/dL. In a converse example, a message is provided whenever a blood glucose reading at any time in the preceding 3 days is above a first predetermined threshold, such as, for example 180 mg/dL or any value from 140 to 500 mg/dL.

Assuming that the results from the measurement of screen 702 would result in a value below a second predetermined threshold (e.g., 70 mg/dL) in the last three days then the message header 706 of "Low Trend" is displayed at screen 704, along with the actual result of the measurement from screen 702. Actuation of the OK button would result in screen 708 which would provide a message 710 relating to the fact that in each of the last 3 days (or any selected time period), the user had a result below the second predetermined threshold. The user can further find additional details by pressing the OK button, which would take the user to screen 712, which provides for a breakdown 714 (e.g., date, time and measured value) of the occurrence of the data meeting the low blood glucose threshold. Similar to Figure 6, where flagging by the user has been enabled in the set up of the user interface, the user interface 700 is configured to prompt the user at screen 604' to enter a tag or flag by highlighting the query 615 in screen 604' which would take the user to screen 605 to allow the user to select and add the appropriate flag (e.g., fasting, before meal, after meal, bedtime, or no flag) 619. After the appropriate flag has been added, the screen returns to screen 604'. Thereafter, the user interface continues from screen 604' to screen 708 and screen 712.

Assuming that the blood glucose result of screen 702 would be higher than the first predetermined high threshold, the user interface 700 would cause screen 720 to display the actual measured result along with a message header 722 highlighted. Actuation of the OK button would result in screen 724 which would provide a message 726 relating to the fact that in each of the last 3 days (or any selected time period), the user had a result above a first predetermined threshold. The user can further find additional details by pressing the OK button which would take the user to screen 728 which provides for a breakdown 730 (e.g., date, time and measured value) of the occurrence of the data above the high blood glucose threshold. Where flagging by the user has been enabled in the set up of the user interface, the user interface 700 is configured to prompt the user at screen 604' to enter a tag or flag by highlighting the query 615 in screen 604' which would take the user to screen 605 to allow the user to select and add the appropriate flag (e.g., fasting, before meal, after meal, bedtime, or no flag) 619.

By virtue of various embodiments described and illustrated herein, applicants have implemented the various user interfaces along with methods of operating a data management unit to provide relevant facts to the user regarding the user's blood glucose data without the user having to perform the analysis himself or herself. It is further believed that the various embodiments described herein provide for relevant information that could be used by the user in discussion with a caretaker or health care provider or even for the user to make adjustments to the user's diabetes management plan. In particular, one method of providing relevant factual information to the user or health care provider can be achieved by: measuring with the analyte sensor for at least one analyte present in each of a plurality of physiological samples taken over a first predetermined time period; storing analyte values representative of the at least one analyte present in each of the plurality of physiological samples; determining whether a number of the stored analyte values over the first predetermined time period meets a minimum threshold; presenting, upon completion of a current analyte measurement and achievement of at least the minimum threshold of the number of stored analyte values, at least one of two selectable message headers that includes at least (a) review of the stored analyte values over the predetermined time period; or (b) trend pattern of the stored analyte values; revealing, upon selection of the message header for review of stored analyte values, a message indicative of a distribution of stored analyte values over at least one of a predetermined range, a first threshold or a second threshold higher than the first threshold; and manifesting, upon selection of the message header for trend pattern, a message indicative of a trend of the stored values over a second predetermined time period. Instead of the providing of the message, the method may include annunciating, upon selection of the message header for review of stored analyte values, a message indicative of a distribution of stored analyte values over at least one of a predetermined range, a first threshold or a second threshold higher than the first threshold; and annunciating, upon selection of the message header for trend pattern, a message indicative of a trend of the stored values over a second predetermined time period.

It is noted that the measuring step in the method may include depositing a physiological sample on a test strip coupled to the analyte meter and initiating a reaction between the analyte in the sample and an enzyme disposed on the test strip, the storing may include indexing, flagging or tagging the stored analyte value to at least one of a time and date at which the measuring was performed, the determining may include defining a range of days within the first predetermined time period and defining the number of measurements as the minimum threshold within the first predetermined time period. The range may include a range selected from a group consisting essentially of three, five, seven, fourteen, thirty, ninety, or one-hundred twenty days. The message indicative of a distribution of stored analyte values may include an identification 608 of a percentage of stored analyte values within a first distribution range, a percentage of stored analyte values, if any, lower than a minimum in the first distribution range and a percentage, if any, higher than a maximum in the first distribution range. Alternatively, the message indicative of a distribution of stored analyte values may include a first distribution range 614, a second distribution range 612 with a value smaller than a minimum in the first distribution range, and a third distribution range 616 with a value greater than a maximum in the first distribution range, actual number of measurements having values within respective first, second and third distribution ranges, and a percentage of the measurements within respective first, second and third distribution ranges. The message indicative of a distribution of stored analyte values above a first predetermined threshold may include measured values and the dates and times corresponding to the respective measured values. Alternatively, the message indicative of a distribution of stored analyte values below a second predetermined threshold may include measured values and the dates and times corresponding to the respective measured values. In a further alternative, the message indicative of a trend of the stored values over a second predetermined time period may include a text indicating one of an increasing trend or decreasing trend in the measured values of the analyte. In yet another alternative, the message indicative of a trend of the stored values may include a table having the date, time, and measured analyte value at such date and time.

The message delivered to the user is preferably alphanumeric textual data that includes at least a portion of the stored analyte data in the message. The message is preferably manifested, e.g., displayed, announced or both, to the user with only a single key actuation, such as, for example from screen 604 directly to any one of screens 606, 610 or 620 in Figure 6; directly from 704 to any one of 708 or 712 or directly from 720 to any one of screens 724 or 728. Alternatively, the device 100 can be configured to deliver the message without actuation of any user interface button. Preferably, this message is delivered to the user subsequent to the measuring or alternatively, prior to any other messages.

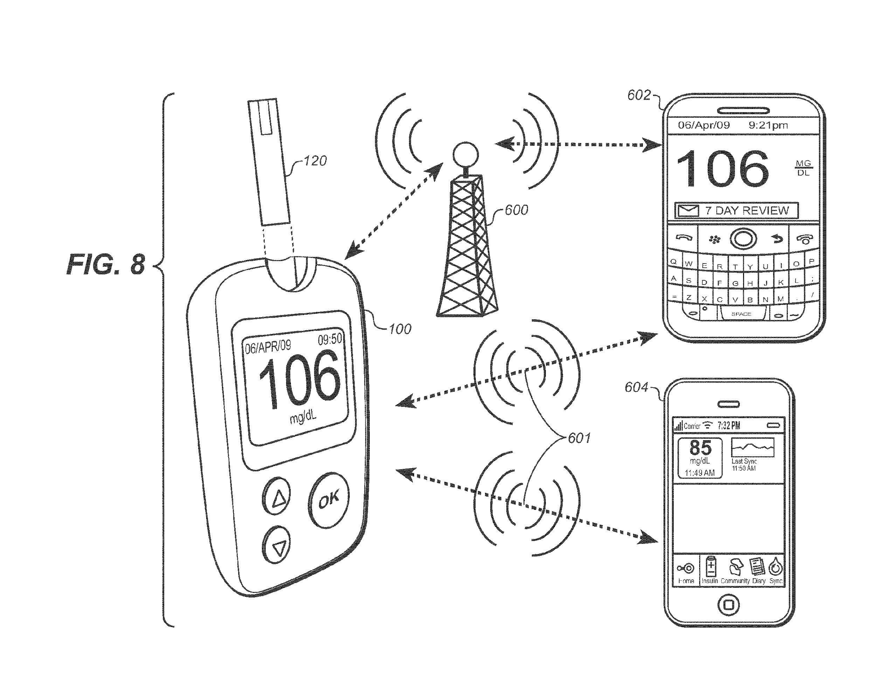

It should be noted that the embodiments of the invention are not limited to messages on the meter 100 but are intended to be replicated across all portable devices capable of wireless communication. It has been observed by applicants that users consider the usage of a medical device, (i.e., a blood glucose meter 100) in public to reflect poorly on the user. However, when such data and messages are replicated or formatted for use on a fashionable mobile communication device, it is believed that users have no hesitation in monitoring their progress on such mobile communication devices. As such, with reference to Figure 8, the meter device 100 is capable of communicating directly to a mobile phone network 600 so as to provide data or messages stored on the meter 100 a variety of mobile communication devices (e.g., cellular phone, smart phones or mobile computing platforms) 602 or 604. Alternatively, the meter 100 can transmit collected data or messages from user interface 400 and 500 to the mobile communication devices 602 or 604 (e.g., mobile phones including smart phones such as Blackberry or iPhone) over a short range network utilizing a suitable protocol 601 such as, for example, BlueTooth, ZigBee, Wi-Fi, or variations and permutations thereof

As noted earlier, the microprocessor can be programmed to generally carry out the steps of various processes described herein. The microprocessor can be part of a particular device, such as, for example, a glucose meter, an insulin pen, an insulin pump, a server, a mobile phone, personal computer, or mobile hand held device. Furthermore, the various methods described herein can be used to generate software codes using off-the-shelf software development tools such as, for example, C, C+, C++, C-Sharp, Visual Studio 6.0, Windows 2000 Server, and SQL Server 2000. The methods, however, may be transformed into other software languages depending on the requirements and the availability of new software languages for coding the methods. Additionally, the various methods described, once transformed into suitable software codes, may be embodied in any computer-readable storage medium that, when executed by a suitable microprocessor or computer, are operable to carry out the steps described in these methods along with any other necessary steps.

While preferred embodiments of the present invention have been shown and described herein, it will be obvious to those skilled in the art that such embodiments are provided by way of example only. For example, the invention can be applied not only to docking stations and glucose meters, but can also be applied to any electronic device that needs a power supply and that can be re-set such as insulin infusion pump, continuous glucose monitoring system and the like. Numerous variations, changes, and substitutions will now occur to those skilled in the art without departing from the invention. Various alternatives to the embodiments of the invention described herein may be employed in practicing the invention. It is intended that the following claims define the scope of the invention and that methods and structures within the scope of these claims and their equivalents be covered thereby.

The following embodiments form part of this disclosure:
1. A diabetes management system comprising:
   a blood glucose test strip having an inlet port to receive a blood sample; and
   a blood glucose measurement device having a test strip port configured to receive the test strip and a microcontroller coupled to a memory, display screen, and user interface buttons so that upon completion of a current blood glucose measurement with the test strip, the display screen presents a value of the current blood glucose measurement and at least one of two selectable message headers on the display screen comprising a review of blood glucose values stored in the memory over a predetermined time period, or a trend pattern of the stored blood glucose values.
2. The system of embodiment 1, in which the message indicative of a distribution of stored blood glucose values comprises an identification of a percentage of stored blood glucose values within a first distribution range, a percentage of stored blood glucose values, if any, lower than a minimum in the first distribution range and a percentage, if any, higher than a maximum in the first distribution range.
3. The system of embodiment 1, in which the message indicative of a distribution of stored blood glucose values comprises a first distribution range, a second distribution range with a value smaller than a minimum in the first distribution range, and a third distribution range with a value greater than a maximum in the first distribution range, actual number of measurements having values within respective first, second and third distribution ranges, and a percentage of the measurements within respective first, second and third distribution ranges.
4. The system of embodiment 1, in which the message indicative of a distribution of stored blood glucose values above a first predetermined threshold comprises measured values and the dates and times corresponding to the respective measured values.
5. The system of embodiment 1, in which the message indicative of a distribution of stored blood glucose values below a second predetermined threshold comprises measured values and the dates and times corresponding to the respective measured values.
6. The system of embodiment 1, in which the message indicative of a trend of the stored values over a second predetermined time period comprises a text indicating one of an increasing trend or decreasing trend in the measured values of the blood glucose.
7. The system of embodiment 1, in which the message indicative of a trend of the stored values comprises a table having the date, time, and measured blood glucose value at such date and time.
8. The system of any one of embodiments 1-7, in which the message comprises a textual message.
9. An analyte test measurement device comprising:
   a housing;
   a measurement sensor disposed in the housing and configured to receive an analyte test strip; and
   a microcontroller coupled to a memory, display screen, user interface buttons and the measurement sensor which are mounted to the housing so that upon completion of a current analyte measurement of a physiological sample with an analyte test strip, the display screen presents the current analyte measurement and at least one of two selectable message headers on the display screen comprising a review of analyte values stored in the memory over a predetermined time period or a trend pattern of the stored analyte values.
10. The device of embodiment 9, in which the message indicative of a distribution of stored analyte values comprises an identification of a percentage of stored analyte values within a first distribution range, a percentage of stored analyte values, if any, lower than a minimum in the first distribution range and a percentage, if any, higher than a maximum in the first distribution range.
11. The device of embodiment 9, in which the message indicative of a distribution of stored analyte values comprises a first distribution range, a second distribution range with a value smaller than a minimum in the first distribution range, and a third distribution range with a value greater than a maximum in the first distribution range, actual number of measurements having values within respective first, second and third distribution ranges, and a percentage of the measurements within respective first, second and third distribution ranges.
12. The device of embodiment 9, in which the message indicative of a distribution of stored analyte values above a first predetermined threshold comprises measured values and the dates and times corresponding to the respective measured values.
13. The device of embodiment 9, in which the message indicative of a distribution of stored analyte values below a second predetermined threshold comprises measured values and the dates and times corresponding to the respective measured values.
14. The device of embodiment 9, in which the message indicative of a trend of the stored values over a second predetermined time period comprises a text indicating one of an increasing trend or decreasing trend in the measured values of the analyte.
15. The device of embodiment 9, in which the message indicative of a trend of the stored values comprises a table having the date, time, and measured analyte value at such date and time.
16. The device of any one of embodiments 9-15, in which the identification comprises a message selected from a group consisting essentially of a textual, visual, audio, or combinations thereof
17. A method of providing analyte information of a user via an analyte meter having a microcontroller coupled to a memory, display, user interface buttons, and an analyte sensor, the method comprising:
   measuring with the analyte sensor for at least one analyte present in each of a plurality of physiological samples taken over a first predetermined time period;
   storing analyte values representative of the at least one analyte present in each of the plurality of physiological samples;
   determining whether a number of the stored analyte values over the first predetermined time period meets a minimum threshold;
   presenting, upon completion of a current analyte measurement and achievement of at least the minimum threshold of the number of stored analyte values, at least one of two selectable message headers comprising:
      (a) review of the stored analyte values over the predetermined time period; or
      (b) trend pattern of the stored analyte values;
   revealing, upon selection of the message header for review of stored analyte values, a message indicative of a distribution of stored analyte values over at least one of a predetermined range, a first threshold or a second threshold higher than the first threshold; and
   manifesting, upon selection of the message header for trend pattern, a message indicative of a trend of the stored values over a second predetermined time period.
18. The method of embodiment 17, in which the measuring comprises depositing a physiological sample on a test strip coupled to the analyte meter and initiating a reaction between the analyte in the sample and an enzyme disposed on the test strip; and in which the storing comprises indexing the stored analyte value to at least one of a time and date at which the measuring was performed.
19. The method of embodiment 17, in which the determining comprises defining a range of days within the first predetermined time period and defining the number of measurements as the minimum threshold within the first predetermined time period, in which the range comprises a range selected from a group consisting essentially of three, five, seven, fourteen, thirty, ninety, or one-hundred twenty days.
20. The method of embodiment 17, in which the message indicative of a distribution of stored analyte values comprises an identification of a percentage of stored analyte values within a first distribution range, a percentage of stored analyte values, if any, lower than a minimum in the first distribution range and a percentage, if any, higher than a maximum in the first distribution range, and the message indicative of a distribution of stored analyte values comprises a first distribution range, a second distribution range with a value smaller than a minimum in the first distribution range, and a third distribution range with a value greater than a maximum in the first distribution range, actual number of measurements having values within respective first, second and third distribution ranges, and a percentage of the measurements within respective first, second and third distribution ranges, and the message indicative of a distribution of stored analyte values above a first predetermined threshold comprises measured values and the dates and times corresponding to the respective measured values.
21. The method of embodiment 17, in which the message indicative of a distribution of stored analyte values below a second predetermined threshold comprises measured values and the dates and times corresponding to the respective measured values.
22. The method of embodiment 17, in which the message indicative of a trend of the stored values over a second predetermined time period comprises a text indicating one of an increasing trend or decreasing trend in the measured values of the analyte.
23. The method of embodiment 17, in which the message indicative of a trend of the stored values comprises a table having the date, time, and measured analyte value at such date and time.
24. The method of embodiment 17, in which the revealing or manifesting comprises displaying the message.
25. A method of providing analyte information of a user via an analyte meter having a microcontroller coupled to a memory, display, user interface buttons, and an analyte sensor, the method comprising:
   measuring with the analyte sensor for at least one analyte present in each of a plurality of physiological samples taken over a first predetermined time period;
   storing analyte values representative of the at least one analyte present in each of the plurality of physiological samples;
   determining whether a number of the stored analyte values over the first predetermined time period meets a minimum threshold;
   querying the user, upon completion of a current analyte measurement and achievement of at least the minimum threshold of the number of stored analyte values, to select at least one of two selectable message headers comprising:
      (a) review of the stored analyte values over the predetermined time period; or
      (b) trend pattern of the stored analyte values;
   annunciating, upon selection of the message header for review of stored analyte values, a message indicative of a distribution of stored analyte values over at least one of a predetermined range, a first threshold or a second threshold higher than the first threshold; and
   annunciating, upon selection of the message header for trend pattern, a message indicative of a trend of the stored values over a second predetermined time period.
26. The method of embodiment 25, in which the audiovisual message indicative of a distribution of stored analyte values comprises identification of a percentage of stored analyte values within a first distribution range, a percentage of stored analyte values, if any, lower than a minimum in the first distribution range and a percentage, if any, higher than a maximum in the first distribution range, and in which the audiovisual message indicative of a distribution of stored analyte values comprises a first distribution range, a second distribution range with a value smaller than a minimum in the first distribution range, and a third distribution range with a value greater than a maximum in the first distribution range, actual number of measurements having values within respective first, second and third distribution ranges, and a percentage of the measurements within respective first, second and third distribution ranges.
27. The method of embodiment 26, in which the audiovisual message indicative of a distribution of stored analyte values above a first predetermined threshold comprises measured values and the dates and times corresponding to the respective measured values, in which the audiovisual message indicative of a distribution of stored analyte values below a second predetermined threshold comprises measured values and the dates and times corresponding to the respective measured values, in which the audiovisual message indicative of a trend of the stored values over a second predetermined time period comprises a text indicating one of an increasing trend or decreasing trend in the measured values of the analyte and in which the audiovisual message indicative of a trend of the stored values having the date with time, and measured analyte value at such date and time.
28. The method of any one of embodiments 2, 10, 22, or 26, in which the identification comprises a message selected from a group consisting essentially of a textual, visual, audio, or combinations thereof.
29. A method of providing analyte information of a user via an analyte meter having a microcontroller coupled to a memory, display, user interface buttons, and an analyte sensor, the method comprising:
   measuring with the analyte sensor for at least one analyte present in each of a plurality of physiological samples taken over a first predetermined time period;
   reporting to the user at least one result from the measuring;
   storing analyte values representative of the at least one analyte present in each of the plurality of physiological samples;
   determining whether a number of the stored analyte values over the first predetermined time period meets a minimum threshold;
   upon determining that the number meets the minimum threshold, evaluating the stored analyte values against one or more rules prestored in the meter to define a message having some of the stored data as part of the message to the user; and
   manifesting the message to the user as the first message delivered to the user before any other messages.
30. A method of providing analyte information of a user via an analyte meter having a microcontroller coupled to a memory, display, user interface buttons, and an analyte sensor, the method comprising:
   measuring with the analyte sensor for at least one analyte present in each of a plurality of physiological samples taken over a first predetermined time period;
   reporting to the user at least one result from the measuring;
   storing analyte values representative of the at least one analyte present in each of the plurality of physiological samples;
   determining whether a number of the stored analyte values over the first predetermined time period meets a minimum threshold;
   upon determining that the number meets the minimum threshold, evaluating the stored analyte values against one or more rules prestored in the meter to define a message having some of the stored data as part of the message to the user; and
   manifesting the message to the user subsequent to the measuring.
31. The method of one of embodiment 29 or embodiment 30, in which the message comprises:
   an identification of a percentage of stored analyte values within a first distribution range, a percentage of stored analyte values, if any, lower than a minimum in the first distribution range and a percentage, if any, higher than a maximum in the first distribution range;
   a first distribution range, a second distribution range with a value smaller than a minimum in the first distribution range, and a third distribution range with a value greater than a maximum in the first distribution range, actual number of measurements having values within respective first, second and third distribution ranges, and a percentage of the measurements within respective first, second and third distribution ranges.
32. The method of embodiment 30, in which the message indicative of a distribution of stored analyte values above a first predetermined threshold comprises measured values and the dates and times corresponding to the respective measured values, in which the message indicative of a distribution of stored analyte values below a second predetermined threshold comprises measured values and the dates and times corresponding to the respective measured values, in which the message comprises a trend of the stored values over a second predetermined time period comprises a text indicating one of an increasing trend or decreasing trend in the measured values of the analyte, and in which the message indicative of a trend of the stored values comprises a table having the date, time, and measured analyte value at such date and time.
33. A diabetes management system comprising:
   a blood glucose test strip having an inlet port to receive a blood sample; and
   a blood glucose measurement device having a test strip port configured to receive the test strip and a microcontroller coupled to a memory, display screen, and user interface buttons so that upon completion of a current blood glucose measurement with the test strip, the display screen presents a value of the current blood glucose measurement and at least one of two selectable message headers on the display screen, the message headers comprising a review of blood glucose values stored in the memory over a predetermined time period, or a prompt to tag the current blood glucose measurement.
34. The system of embodiment 33, in which the message indicative of a distribution of stored blood glucose values comprises an identification of a percentage of stored blood glucose values within a first distribution range, a percentage of stored blood glucose values, if any, lower than a minimum in the first distribution range and a percentage, if any, higher than a maximum in the first distribution range, in which the message indicative of a distribution of stored blood glucose values comprises a first distribution range, a second distribution range with a value smaller than a minimum in the first distribution range, and a third distribution range with a value greater than a maximum in the first distribution range, actual number of measurements having values within respective first, second and third distribution ranges, and a percentage of the measurements within respective first, second and third distribution ranges, in which the message indicative of a distribution of stored blood glucose values above a first predetermined threshold comprises measured values and the dates and times corresponding to the respective measured values, in which the message indicative of a distribution of stored blood glucose values below a second predetermined threshold comprises measured values and the dates and times corresponding to the respective measured values, in which the prompt comprises contextual information contemporaneous to the measured value, the information chosen from the group consisting of a fasting condition, a before meal condition, an after meal condition, and a bedtime condition and, in which one of the message headers comprises a query "HOW AM I DOING?"
35. A method of providing analyte information of a user via a glucose meter having a microcontroller coupled to a memory, display, user interface buttons, and a glucose sensor, the method comprising:
   measuring a glucose concentration with the glucose sensor in each of a plurality of physiological samples taken over a first predetermined time period;
   automatically identifying, for a particular day, a low trend in glucose values that is detected on a particular day where at least one glucose concentration is below a second predetermined threshold in each of the last three days;
   automatically identifying, for a particular day, a high trend in glucose values that is detected on a particular day where at least one glucose concentration is above a first predetermined threshold in each of the last three days at a same time interval; and
   reporting to the user a plurality of dates where each date has only one corresponding message, the corresponding messages comprise either the high trend or the low trend, and if on a particular date both the high trend and the low trend are triggered, then only the low trend is reported to the user.
36. The method of embodiment 35, in which the time interval is about three hours, in which the corresponding messages further comprises either the high trend, the low trend, or that a user selected a report prompt; and if on a particular date both the high trend and the low trend are not triggered and the user selected the report prompt, then a message is reported to the user indicating that the report was selected.

## Claims

1. A method of providing analyte information of a user via a glucose meter having a microcontroller coupled to a memory, display, user interface buttons, and a glucose sensor, the method comprising:
measuring a glucose concentration with the glucose sensor in each of a plurality of physiological samples taken over a first predetermined time period;
automatically identifying, for a particular day, a low trend in glucose values that is detected on a particular day where at least one glucose concentration is below a second predetermined threshold in each of the last three days;
automatically identifying, for a particular day, a high trend in glucose values that is detected on a particular day where at least one glucose concentration is above a first predetermined threshold in each of the last three days at a same time interval; and
reporting to the user a plurality of dates where each date has only one corresponding message, the corresponding messages comprise either the high trend or the low trend, and if on a particular date both the high trend and the low trend are triggered, then only the low trend is reported to the user.

2. The method of claim 1, in which the time interval is about three hours, in which the corresponding messages further comprises either the high trend, the low trend, or that a user selected a report prompt; and if on a particular date both the high trend and the low trend are not triggered and the user selected the report prompt, then a message is reported to the user indicating that the report was selected.
